# EUROPEAN PATENT SPECIFICATION

(11) **EP 0 581 550 B1**
(45) Date of publication and mention of the grant of the patent: **17.12.1997**
(21) Application number: 93305859.6
(22) Date of filing: 23.07.1993
(51) Int. Cl.: C01B 11/02

(54) **Solid compositions capable of releasing chlorine dioxide**
Chlordioxyd freisetzende feste Zusammensetzungen
Compositions solides capables de libérer du dioxyde de chlore

(30) Priority: 23.07.1992 IL 102627
(43) Date of publication of application: 02.02.1994
(73) Proprietor: Marzouk, Yosef, Tel-Aviv 69364 (IL); ABIC LIMITED, Natanya (IL)
(72) Inventor: Marzouk, Yosef, Tel-Aviv 69364 (IL); Gutman, Yakov, Holon 58847 (IL)
(74) Representative: Sanderson, Laurence Andrew

(56) References cited:
- DE-A- 2 712 574
- US-A- 4 547 381
- DATABASE WPI Week 8847, Derwent Publications Ltd., London, GB; AN 88-333818

## Description

The present invention relates to solid compositions capable of releasing chlorine dioxide very quickly on dissolution in water.

Chlorine dioxide (ClO₂) is an oxychlorine compound which is gaseous at room temperature and normal pressure, and it is of course well-known and widely used for a number of applications.

Thus in its capacity as an oxidizing agent it is well-accepted for bleaching pulp in the paper industry, for bleaching fabrics, for the oxidative deodorization of malodorous substances in water and waste water, and indeed for some other industrial uses. Moreover, in its capacity as a source of chlorine it can advantageously by used as a biocide and sanitizer, replacing chlorine itself in the treatment of potable water and more generally as a disinfectant or antiseptic.

The even more widespread use of chlorine dioxide is however restricted by two factors:
1. Chlorine dioxide cannot be stored and transported as a gas, while its solubility in water is limited, and the solution is unstable; while anyway
2. Chlorine dioxide as a gas is very toxic, with a specific effect on the respiratory tract.

In view of the difficulties involved in the preparation of chlorine dioxide and the dangers attached in its storage and transportation, on the whole its use is therefore confined to installations where the production of ClO₂ can take place "on-site", thus located near the point-of-use.

The known manufacturing processes for chlorine dioxide are based on one or other of two source substances, namely either sodium chlorite or sodium chlorate. In all the known processes it is necessary to add a second reactant in great excess to one or other of the above-mentioned source substances, in order thereby to displace the reaction equilibrium to such a degree as to achieve the maximum yield of chlorine dioxide in a minimum of time. Thus for instance in one particular process the addition of an excess of lactic acid (which is an organic acid) to a solution of sodium chlorite (NaClO₂) shifts the equilibrium between chlorine dioxide and chlorous acid in that solution and leads to the evolution of chlorine dioxide, but the yield of the latter is always far short of 100%.

Against the background outlined above, clearly there is a great and long-standing need for a solid composition which would be safe to manufacture, store, transport and use, and yet be capable of very quickly releasing chlorine dioxide upon dissolution in water, with a yield approaching 100%, utilizing only a minimal excess of reagents and equally yielding only minimal amounts of by-products, e.g. chlorine, chlorous acid, chloric acid, etc.

Solid compositions (for use as slow-release disinfection and sterilizing agents) were disclosed many years ago, for instance in DE-A-2,712,574, which when dissolved in water are capable of generating chlorine dioxide by a reaction involving sodium chlorite and sodium dichloro-s-triazinetrione (or sodium dichloroisocyanurate, Na-DCC) but these components were there used in widely-differing molar ratios and buffered to react at weakly-acidic or even alkaline pH values - with the result, as we have now recognized, that the evolution of chlorine dioxide is slow (as indeed was probably intended) with formation of by-products and anyway very far from complete.

According to the present invention there is provided a solid composition comprising:
(a) a chlorite salt;
(b) an oxidizing chlorine-releasing agent in the form of one or more sodium- and/or potassium- dichloro-s-triazinetrione(s) and/or -trichloro-s-triazinetrione(s); and
(c) a proton-donor serving as a water-soluble agent capable of lowering the pH of the aqueous solution to less than 3;
and in which composition the stoichiomolecular ratio of components (a) : (b) : (c) is substantially 4 : 1 : 3.

There are a number of known oxidizing chlorine-releasing salts which are capable of use in the compositions of this invention, including for instance trichloro isocyanurate, but in our experience the most effective such compound, which therefore is preferred, is dichloro isocyanurate (Na-DCC).

Suitable proton-donors include, for example, a variety of inorganic acid salts, such as sodium- or potassium-hydrogen sulphates or pyrosulphates, as well as certain organic acids, e.g. citric acid, malic acid, (racemate or optical antipode), etc.

When the various specified components, namely chlorite salt (a), oxidising chlorine-releasing agent (b) and proton-donor (c), are present in the composition of this invention in substantially the specified molar ratios:$\text{(a) : (b) : (c) = 4 : 1 : 3}$ then the reaction proceeds in accordance with the following chemical equation:

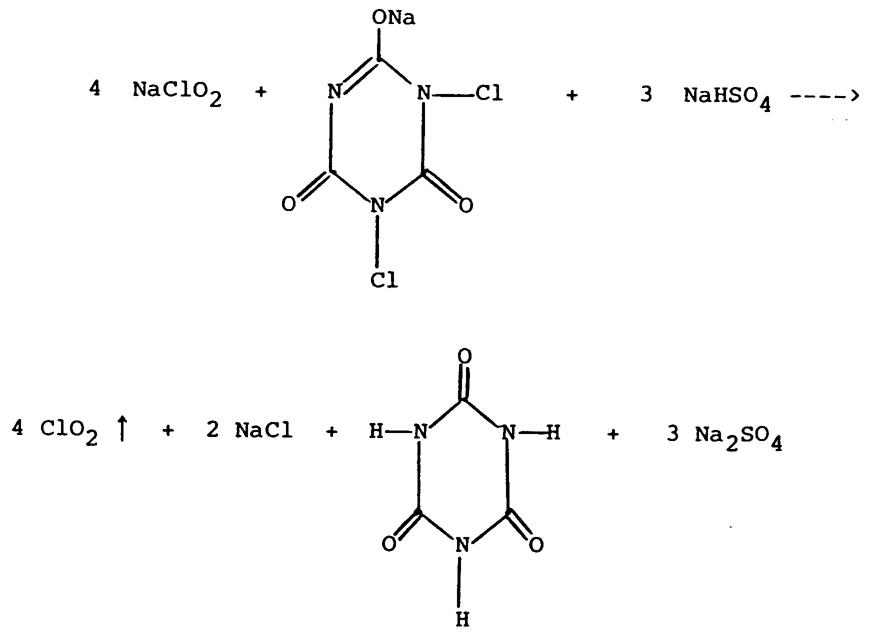

The proton-donor NaHSO₄ may of course be present in a small excess.

The invention includes a process for the preparation of the composition according to the present invention, in which the above-specified dry components are brought together and thoroughly admixed under substantially anhydrous conditions.

It also extends to a chlorine-dioxide-containing aqueous solution formed by dissolving the solid composition in water. On being dissolved in a suitable amount of water the composition according to the present invention evolves chlorine dioxide in a yield approaching 100%. Moreover, the release of chlorine dioxide is surprisingly fast, and the equilibrium of the reaction shown above is thereby irreversibly displaced to the right, yielding the desired product in more or less the strict stoichiomolecular ratio. Furthermore, while of course we cannot rule out all possibility that the reaction might sometimes give rise to the kind of by-products obtained in similar reactions (e.g. chlorine, chlorous acid and chloric acid) in our experience that does not happen, at least within the limits of our analytical determinations.

According to another aspect of the present invention there is also provided a process for the production of chlorine dioxide in which a composition as herein disclosed is dissolved in a suitable amount of water.

As already indicated above chlorine dioxide in aqueous solution is well-known to be an excellent biocide, virucide, bactericide, fungicide, algaecide and sporocide. It is able to exert its effect on such microorganisms after only a short period of contact, usually a matter of merely minutes. The presence of proteins or other contaminating substances does not inhibit its effect on the microorganisms. The use of chlorine dioxide in aqueous solution also seems to be free from any danger of an halogenation reaction occurring.

Thus the composition and the process according to the present invention provide means for releasing chlorine dioxide immediately, easily and safely, under controlled conditions. As the reaction is fast and nearly complete, the toxic side-effects of any residual by-products are restricted to a minimum - and this enables the use of the composition as an antiseptic for skin, mucous membranes and even in body cavities.

In yet another aspect of this invention there are provided antiseptic and sanitizing preparations containing the solid composition and/or aqueous solution thereof, optionally together with other usual additives.

For similar reasons, the compositions of this invention are suitable for use in the treatment of potable water, e.g. as a swimming pool sanitizer and for other water and waste-water treatments.

The amount of water in which the composition can effectively be dissolved can vary very widely, dependent on the kind of use envisaged - and indeed there can be situations in which the humidity of the ambient air may be sufficient to release chlorine dioxide from the solid composition.

In order that the present invention shall be well understood it will be described in more detail, but only by way of illustration, with reference to the following Examples (and Comparative Examples), in which abbreviation "T.G." is used to mean "technical grade", while the abbreviation "C.P." is used to mean "chemically pure".

### EXAMPLE 1

A solid composition in accordance with the invention was prepared by intimately mixing together the following ingredients in the following amounts:

| | |
|---|---|
| Sodium Chlorite (T.G 80%) | 42 g. |
| Na-DCC (T.G. 96%) | 22 g. |
| Sodium Bisulfate (C.P.) | 36 g. |
| | $\overline{\text{100 g.}}$ |

1 Gram of this solid composition was then dissolved in 99 grams of water, yielding a yellowish-green solution containing approximately 0.25% of chlorine dioxide. This corresponds to a yield of approximately 100%, based on the sodium chlorite.

### EXAMPLE 2

A solid composition in accordance with the invention was prepared by intimately mixing together the following ingredients in the following amounts:

| | |
|---|---|
| Sodium Chlorite (T.G. 80%) | 42 g. |
| Na-DCC (T.G. 96%) | 22 g. |
| Sodium Pyrosulphate (C.P.) | 36 g. |
| | $\overline{\text{100 g.}}$ |

1 Gram of this solid composition was dissolved in 99 grams of water, yielding a yellowish-green solution containing approximately 0.25% of chlorine dioxide. This corresponds to a yield of approximately 100% based on the sodium chlorite.

### EXAMPLE 3

A solid composition in accordance with the invention was prepared by intimately mixing together the following ingredients in the following amounts:

| | |
|---|---|
| Sodium Chlorite (T.G. 80%) | 40.3 g. |
| Na-DCC (T.G. 96%) | 20.7 g. |
| Potassium Bisulphate (C.P.) | 39.0 g. |
| | $\overline{\text{100.0 g.}}$ |

1 Gram of this solid composition was dissolved in 99 grams of water, yielding a yellowish-green solution containing approximately 0.24% of chlorine dioxide. This corresponds to a yield of approximately 100% based on the sodium chlorite.

### EXAMPLE 4

A solid composition in accordance with the invention was prepared by intimately mixing together the following ingredients in the following amounts:

| | |
|---|---|
| Sodium Chlorite (T.G. 80%) | 40.3 g. |
| Na-DCC (T.G. 96%) | 20.7 g. |
| Potassium Pyrosulphate (C.P.) | 39.0 g. |
| | $\overline{\text{100.0 g.}}$ |

1 Gram of this solid composition was dissolved in 99 grams of water, yielding a yellowish-green solution containing approximately 0.24% chlorine dioxide. This corresponds to a yield of approximately 100% based on the sodium chlorite.

### EXAMPLE 5 (Comparative)

Merely for purposes of comparison (especially with Examples 6 and 7 below) a solid composition free from Na-DCC (or any other oxidizing chlorine-releasing salt) and therefore outside the scope of this invention was prepared by intimately mixing together the following ingredients in the following amounts:

| | |
|---|---|
| Sodium Chlorite (T.G. 80%) | 42.5 g. |
| Sodium Bisulphate (C.P.) | 50.0 g. |
| Sodium Chloride (C.P.) | 7.5 g. |
| | $\overline{\text{100.0 g.}}$ |

This composition was dissolved in water, whereupon the evolution of chlorine dioxide started and then continued slowly for a few days. Immediately following dissolution the yield of chlorine dioxide was found to be 7-8%; after 30 minutes the yield had increased to 30%; and at the end of 3 days the final yield obtained had reached 63%.

### EXAMPLE 6 (Comparative)

Again for purposes of comparison (especially with Example 5 above and Example 7 below) a solid composition free from any proton-donor and therefore outside the scope of this invention was prepared by intimately mixing together the following ingredients in the following amounts:

| | |
|---|---|
| Sodium Chlorite (T.G. 80%) | 49.0 g. |
| Na-DCC (T.G. 96%) | 51.0 g. |
| | $\overline{\text{100.0 g.}}$ |

This composition was dissolved in water, and thereupon a very slow release of chlorine dioxide could be detected - but initially it measured only 1%, and after 30 minutes it was still only 5%, while even after 3 hours it had attained no more than 30%. Some release of oxygen was also observed.

### EXAMPLE 7

A solid composition in accordance with this invention (although a somewhat less-preferred one, containing trichloroisocyanurate instead of Na-DCC), was prepared (especially for purposes of contrast with Comparative Examples 5 and 6 above) by intimately mixing together the following ingredients in the following amounts:

| | |
|---|---|
| Sodium Chlorite (T.G. 80%) | 42.0 g. |
| Trichloroisocyanurate (C.P.) | 15.0 g. |
| Sodium Bisulphate (C.P.) | 43.0 g. |
| | $\overline{\text{100.0 g.}}$ |

This composition was dissolved in water. As compared with the more-preferred compositions of Example 1 - 4, it was clear that the replacement of Na-DCC by trichloroisocyanurate slowed down the rate of release of the chlorine dioxide - but, even so, it was found that the release of chlorine dioxide had attained about 100% within 10-15 minutes.

### EXAMPLE 8

A solid composition in accordance with this invention was prepared by intimately mixing together the following ingredients in the following amounts:

| | |
|---|---|
| Sodium Chlorite (T.G. 80%) | 42 g. |
| Na-DCC (T.G. 96%) | 22 g. |
| Citric Acid (C.P.) | 36 g. |
| | $\overline{\text{100 g.}}$ |

1 Gram of this solid composition was dissolved in 99 grams of water, yielding a yellowish-green solution containing approximately 0.25% chlorine dioxide. This corresponds to a yield of approximately 100% based on the sodium chlorite.

### EXAMPLE 9

A solid composition in accordance with this invention was prepared by intimately mixing together the following ingredients in the following amounts:

| | |
|---|---|
| Sodium Chlorite (T.G. 80%) | 42 g. |
| Na-DCC (T.G. 96%) | 22 g. |
| D.L-Malic Acid (C.P.) | 36 g. |
| | $\overline{\text{100 g.}}$ |

1 Gram of this solid composition was dissolved in 99 grams of water, yielding a yellowish-green solution containing approximately 0.25% chlorine dioxide. This corresponds to a yield of approximately 100% based on the sodium chlorite.

## Claims

1. A solid composition capable of releasing chlorine dioxide upon dissolution in water, said composition comprising:
(a) a water-soluble chlorite salt;
(b) an oxidizing chlorine-releasing agent, in the form of one or more sodium- and/or potassium- dichloro-s-triazinetrione(s)s and/or trichloro-s-triazinetrione(s); and
(c) a proton-donor serving as a water-soluble agent capable of lowering the pH of an aqueous solution to less than 3;
and in which composition the stoichiomolecular ratio of components (a) : (b) : (c) is substantially 4 : 1 : 3.

2. A solid composition as claimed in claim 1, in which the chlorite salt (a) is or includes the sodium- and/or potassium salt(s) thereof.

3. A solid composition as claimed in claim 1 or claim 2, in which the proton-donor (c) is or includes one or more sodium- and/or potassium- hydrogen sulphate(s) and/or pyrosulphate(s).

4. A solid composition as claimed in claim 1 or claim 2, in which the proton-donor is or includes citric acid and/or malic acid.

5. A solid composition as claimed in any of the preceding claims, in which the proton-donor (c) is present in a slight stoichiomolecular excess relative to the other components.

6. A chlorine-dioxide-containing aqueous solution formed by dissolving a solid composition as claimed in any of the preceding claims in water.

7. A solution as claimed in claim 6, in which the composition has been dissolved in a concentration of the order of 1% by weight.

8. Antiseptic and sanitizing preparations containing as biocidally-active ingredient therein a solid composition and/or an aqueous solution as claimed in any of the preceding claims optionally together with other usual additives appropriate to their function.

9. In an industrial and/or water-treatment process utilizing chlorine dioxide the step of generating said chlorine dioxide by dissolving a solid composition as claimed in any of claims 1 to 5 in water extemporaneously in situ within the processing installation.

## Patentansprüche

1. Feste Zusammensetzung, die beim Lösen in Wasser Chlordioxid freisetzen kann, umfassend:
(a) ein wasserlösliches Chlorit-Salz
(b) ein Chlor freisetzendes Oxidationsmittei in Form eines oder mehrerer Natrium- und/oder Kaliumdichlor-s-triazintrions(e) und/oder Trichlor-s-triazintrions(e), und
(c) einen Protonen-Donor, der als wasserlösliches Mittel dient, welcher den pH einer wäßrigen Lösung auf weniger als 3 erniedrigen kann;
und worin in der Zusammensetzung das stöchiometrische Verhältnis der Komponenten (a) : (b) : (c) im wesentlichen 4 :1 : 3 beträgt.

2. Feste Zusammensetzung gemäß Anspruch 1, worin das Chloritsalz (a) das (die) Natrium- und/oder Kaliumsalz(e) ist oder einschließt.

3. Feste Zusammensetzung gemäß Anspruch 1 oder Anspruch 2, worin der Protonen-Donor (c) ein oder mehrere Natrium- und/oder Kaliumhydrogensulfat(e) und/oder -pyrosulfate(e) ist oder einschließt.

4. Feste Zusammensetzung gemäß Anspruch 1 oder Anspruch 2, worin der Protonen-Donor Citronensäure und/oder Äpfelsäure ist oder einschließt.

5. Feste Zusammensetzung gemäß irgendeinem der vorhergehenden Ansprüche, worin der Protonen-Donor (c) in einem geringen stöchiometrischen Überschuß in bezug auf die anderen Komponenten vorliegt.

6. Chlordioxid enthaltende wäßrige Lösung, die durch Lösen einer festen Zusammensetzung gemäß irgendeinem der vorhergehenden Ansprüche in Wasser gebildet wird.

7. Lösung gemäß Anspruch 6, worin die Zusammensetzung in einer Konzentration in der Größenordnung von 1 Gew.-% gelöst wird.

8. Antiseptische und keimfrei machende Präparate, die als biozidisch wirksamen Bestandteil darin eine feste Zusammensetzung und/oder eine wäßrige Lösung gemäß irgendeinem der vorhergehenden Ansprüche, gegebenenfalls zusammen mit anderen brauchbaren, für deren Funktion geeigneten Additiven, enthalten.

9. Die Stufe der Erzeugung dieses Chlordioxids in einem Chlordioxid verwendenden, gewerblichen Verfahren und/oder Wasserbehandlungsverfahren, indem man eine feste Zusammensetzung gemäß irgendeinem der Ansprüche 1 bis 5 auf improvisierte Weise in situ in der Verarbeitungsanlage in Wasser löst.

## Revendications

1. Composition solide capable de libérer du dioxyde de chlore après dissolution dans l'eau, ladite composition comprenant:
(a) un chlorite hydrosoluble;
(b) un agent libérant du chlore oxydant, sous forme d'une ou plusieurs dichloro-s-triazinetriones et/ou trichloro-s-triazinetrione sodiques et/ou potassiques ; et
(c) un donneur de protons servant d'agent hydrosoluble capable d'abaisser le pH d'une solution aqueuse à moins de 3;
et composition dans laquelle le rapport stoechiométrique des composants (a):(b):(c) est sensiblement 4:1:3.

2. Composition solide selon la revendication 1, dans laquelle le chlorite (a) est ou inclut un ou plusieurs de ses sels de sodium et/ou de potassium.

3. Composition solide selon la revendication 1 ou 2, dans laquelle le donneur de protons (c) est ou inclut un ou plusieurs hydrogénosulfates et/ou un ou plusieurs pyrosulfates de sodium et/ou de potassium.

4. Composition solide selon la revendication 1 ou 2, dans laquelle le donneur de protons est ou inclut l'acide citrique et/ou l'acide malique.

5. Composition solide selon l'une quelconque des revendications précédentes, dans laquelle le donneur de protons (c) est présent en un léger excès stoechiomoléculaire par rapport aux autres composants.

6. Solution aqueuse contenant du dioxyde de chlore formée en dissolvant une composition solide selon l'une quelconque des revendications précédentes dans l'eau.

7. Solution selon la revendication 6, dans laquelle la composition a été dissoute en une concentration de l'ordre de 1% en poids.

8. Préparations antiseptiques et sanitaires contenant en tant qu'ingrédient biacide actif dans une composition solide et/ou une solution aqueuse selon l'une quelconque des revendications précédentes, éventuellement conjointement avec d'autres additifs habituels appropriés à leur fonction.

9. Dans un procédé industriel et/ou de traitement des eaux utilisant le dioxyde de chlore, étape consistant à engendrer ledit dioxyde de chlore en dissolvant extemporanément une composition solide selon l'une quelconque des revendications 1 à 5 dans l'eau in situ dans l'installation du traitement.
